(19) Europäisches Patentamt / European Patent Office / Office européen des brevets

(11) **EP 3 639 675 A1**

(12) **EUROPEAN PATENT APPLICATION**

(43) Date of publication:
**22.04.2020 Bulletin 2020/17**

(21) Application number: **18201536.2**

(22) Date of filing: **19.10.2018**

(51) Int Cl.:
*A23K 10/10* (2016.01)   *A23K 50/75* (2016.01)
*A23K 50/30* (2016.01)   *A23K 50/40* (2016.01)
*A23K 50/80* (2016.01)   *A23K 50/60* (2016.01)
*A61K 31/7028* (2006.01)   *A61K 31/7032* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **EW Nutrition GmbH**
**49429 Visbek (DE)**

(72) Inventors:
• **Awati, Ajay**
**Swindon SN5 6NQ (GB)**
• **Borchers, Thilo**
**49377 Vechta (DE)**
• **Thole, Karin**
**49661 Cloppenburg (DE)**
• **Tepe, Daniel**
**26122 Oldenburg (DE)**

(74) Representative: **Michalski Hüttermann & Partner Patentanwälte mbB**
**Speditionstraße 21**
**40221 Düsseldorf (DE)**

(54) **RHAMNOLIPID COMPOSITIONS, AN ANIMAL FEED OR ADDITIVE COMPRISING THE COMPOSITIONS, A METHOD OF FEEDING MONOGASTRIC ANIMALS AND THE USE OF THE COMPOSITION FOR DIGESTIBILITY AND/OR ABSORPTION ENHANCEMENT**

(57)    The present invention relates to compositions comprising one or more rhamnolipids for supplementation of a monogastric animal diet. The invention further relates to animal feed or feed additive comprising said composition, and to the use of said composition as a digestibility enhancer and/or absorption enhancer in monogastric animal fee.

The monogastric animal may be selected from the group consisting of poultry, preferably chicken, turkey and duck, pig, fish, shrimp, dog, cat, horse, pre-ruminant calf.

At least one rhamnolipid comprises one or two β-hydroxy fatty acid units, preferably wherein at least one of the β-hydroxy fatty acid units is β-hydroxydecanoic acid and at least one of the β-hydroxy fatty acid units is based on a fatty acid chain length of 8 to 16 carbon atoms and said at least one β-hydroxy fatty acid unit contains no, one or two double bonds.

According to the use, said composition is dosed at 10 mg/kg to 710 mg/kg in the animal feed.

Fig. 1

Printed by Jouve, 75001 PARIS (FR)

## Description

### Field of the invention

The present application relates to compositions comprising one or more rhamnolipids for supplementation of a monogastric animal diet. The invention further relates to animal feed or feed additive comprising said composition, and to the use of said composition as a digestibility enhancer and/or absorption enhancer in monogastric animal feed.

### Background

Rhamnolipids are glycolipids containing a hydrophilic group, consisting of either one or two (L)-rhamnose molecules, with a glycosidic linkage to the hydrophobic group made up of one or two β-hydroxy fatty acids. Specifically, there are two main classes of rhamnolipids, containing one and two rhamnose molecules, which are known as "mono-rhamnolipids" (compound I, see below) and "di-rhamnolipids" (compound II, see below), respectively (Chong and Li, 2017). Rhamnose is a naturally occurring deoxy sugar; it can be classified as either a methyl-pentose or a 6-deoxy-hexose. Rhamnose occurs in nature in its L-form as L-rhamnose (6-deoxy-L-mannose), and is commonly bound to other sugars in nature.

Rhamnolipids are a class of glycolipid produced by *Pseudomonas aeruginosa* and various other microorganisms. The glycosyl head group, the rhamnose moiety, is coupled to a 3-(hydroxyalkanoyloxy)alkanoic acid (HAA) fatty acid tail, such as, *e.g.*, 3-hydroxydecanoic acid.

I                                                    II

Microbial fermentation produces a diversity of rhamnolipid congeners with variations in the chain lengths, degree of unsaturation for the fatty acid chains, degree of branching of the HAA moiety, and differences in the number of rhamnose molecules. Rhamnolipid composition varies with the growth media used and the environmental conditions. It has been estimated that approximately 60 rhamnolipid congeners and homologues exist in the fermentation broth of respective microbial cultures, with predominant rhamnolipid species and the concentrations of the congeners being dependent on the rhamnolipid-producing strains (Chong and Li, 2017).

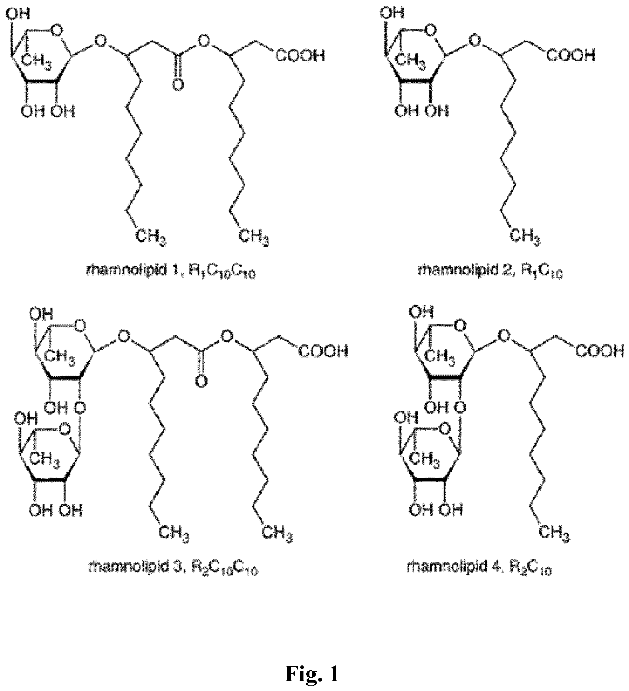

Rhamnolipids were initially found as exoproducts of the opportunistic pathogen *Pseudomonas aeruginosa* and described as a mixture of four congeners: α-L-rhamnopyranosyl-α-L-rhamnopyranosyl-β-hydroxydecanoyl-β-hydroxydecanoate (Rha-Rha-C10-C10), α-L-rhamnopyranosyl-α-L-rhamnopyranosyl-β-hydroxydecanoate (Rha-Rha-C10), as well as their mono-rhamnolipid congeners Rha-C10-C10 and Rha-C10 (Abdel-Mawgoud *et al.,* 2010). The structure of these four congeners is depicted in Fig. 1.

*Pseudomonas aeruginosa* is still the most competent producer of rhamnolipids. The three key enzymes for rhamnolipid biosynthesis, RhlA, RhlB and RhlC, are found almost exclusively in *Pseudomonas* sp. and *Burkholderia* sp., in particular in *P. aeruginosa*, *Burkholderia thailandensis* and *Burkholderia pseudomallei*, but have also been successfully expressed in several non-pathogenic host bacteria to produce rhamnolipids in large scales. The composition of mono- and di-rhamnolipids can also be modified through altering the expression levels of RhlB and RhlC. In addition, cell-free rhamnolipid synthesis by using the key enzymes and precursors from non-pathogenic sources is thought to not only eliminate pathogenic effects of the producer cells and simplify the downstream purification processes, but also to circumvent the complexity associated with the rhamnolipid biosynthesis (Chong and Li, 2017; Irorere *et al.,* 2017).

Rhamnolipids are frequently cited as the best characterized bacterial surfactants. Surfactants are chemical compounds that display surface activity. They have predilection for interfaces of dissimilar polarities (liquid-air or liquid-liquid) and are soluble in both organic (non-polar) and aqueous (polar) solvents. This property originates from their amphiphilic (or amphipathic) structures, which comprise both hydrophilic (head) and hydrophobic groups (tail).

Biosurfactants are surfactants of biological origin and have attracted much attention because they represent ecological alternatives to their synthetic counterparts. They can exhibit lower toxicity, potentially high activities, and stability at extremes of temperature, pH, and salinity. Furthermore, they are biodegradable, making them environmentally friendly. The hydrophilic part of biosurfactants is usually composed of sugars, amino acids, or polar functional groups like carboxylic acid groups, the hydrophobic part is typically an aliphatic hydrocarbon chain of β-hydroxy fatty acids. Surfactants are generally classified according to the charge carried by their polar groups (head) into cationic, anionic, amphoteric, and nonionic (Abdel-Mawgoud *et al.*, 2010).

Rhamnolipids possess unique high surface activity and thus have been extensively gained interests in a wide variety of industrial applications (Ali *et al.*, 2017). Rhamnolipid-producing microorganisms have been exploited for microbial enhanced oil recovery (MEOR) by aiding with increasing yields from oil reservoirs. Addition of rhamnolipids or rhamnolipid-containing cell-free broth are effective in inhibiting growth of phytopathogens and serve as biocontrol agents against several phytopathogenic fungi for crop protection. In medical use rhamnolipids are attractive as biofilm control agents to prevent medical device-related infections. De Santo (US 7,985,722 B2) relates to rhamnolipid-based formulations to clean, disinfect, deodorize, and act as an antimicrobial and antifungal agent for wound treatment in animals. Rhamnolipids also have been used as structure-directing agents in nanoparticle synthesis (Chong and Li, 2017). Furthermore, rhamnolipid-based biosurfactants have been used for environmental aspects such as soil and ground water remediation, and for removal of petroleum hydrocarbon and heavy metal contaminants (Liu *et al.* 2018).

In food processing, rhamnolipids can serve as antimicrobial agents preventing food spoilage and for sanitization; rhamnolipids inhibit the growth of foodborne pathogenic bacteria, like *e.g.*, *L. monocytogenes*, *B. subtilis.* Rhamnolipids also prevent the formation of biofilms due to their anti-adhesive nature (Vatsa *et al.*, 2010; Magalhaes and Nitschke, 2013). In addition to extending food shelf life by inhibiting mold and bacterial growth, particularly of gram-positive and spore-forming bacteria, rhamnolipids have been found to improve texture of starch-containing products, control the agglomeration of fat globules, and improve stability, consistency and texture of oils and fat-based products and inhibits separation (Sinumvayo and Ishimwe, 2015).

Although rhamnolipids offer the advantages of having relatively high surface activities and being produced in high yields after relatively short incubation periods by well-understood, easy to cultivate microorganism, on the other hand, they are considered one of the virulence aspects contributing to the pathogenesis of *P. aeruginosa* contaminations (Ali *et al.*, 2017). This has necessitated the enigineering of specific strains and the tight control of rhamnolipid biosynthesis pathways.

Khafagy *et al.* (2017) found that rhamnolipids can enhance *in-vivo* oral bioavailability of poorly absorbed drug molecules in rats. Using low doses of rhamnolipids (5 μg/kg) increased intestinal absorption of drug molecules; however, higher concentrations (≥ 12.5 μg/kg) led to a progressive damaging, cytotoxic effect on the rat intestinal tissue with no evident ability of self-healing.

EP 2,074,889 A1 (Idemitsu Kosan Co.) teaches the use of a feed additive comprising mannosylerythritol lipids and/or rhamnolipids for preventing or treating an infectious disease, in particular of an infectious disease caused by a gram-positive bacterium, in birds and mammals. The use of said feed additive was further found to improve fermentation in the rumen of a ruminant animal, and thus to contribute to suppression of the generation of greenhouse gases. It was found out that glycolipids such as mannosylerythritol lipids (MEL) and rhamnolipids (RL) suppress the generation of methane and promote the generation of propionic acid in the rumen of a ruminant animal. The concentrations of acetic acid, butyric acid, isobutyric acid, valeric acid, and isovaleric acid were found to significantly decrease by adding RL and MEL; on the other hand, the concentration of propionic acid was found to significantly increase ruminant animals. That means it is possible to suppress generation of methane and to promote generation of propionic acid, resulting in promotion of growth of the ruminant animals and thus indirectly in an improvement of the feed efficiency.

On the other hand, Damasceno *et al.* (2012) found that the combined use of a rhamnolipid biosurfactant produced by *Pseudomonas aeruginosa* and an enzyme preparation obtained from solid-state fermentation in the anaerobic treatment of an effluent with a high fat content may *enhance* methane production and enable the use of anaerobic technology.

The patent application CN102696880A discloses a biologic emulsifier which is characterized by being prepared by two or more than two of sophorolipid, rhamnolipid and trehalose. Said biologic emulsifier was found to be able to improve livestock fat digestion utilizing rate. However, the impact of rhamnolipids or rhamnolipid comprising compositions as feed additives on non-ruminant, monogastric animals, in particular with regard to various types of nutrients apart from fat, has not been studied so far.

Animal feed expenditures are one of the major cost factors in live-stock farming and animal husbandry. For maintaining and improving animal health and nutrition conditions in modern live-stock farming at affordable costs, there is a high need for the development of improved feeding modalities and nutrient compositions, including the identification of beneficial feed additives.

Whereas ruminant herbivores digest cellulose and other fibre nutrients by microbial fermentation, monogastric animals (monogastrics) cannot digest the cellulose and other fiber molecules as efficiently as ruminants.

It is hence one object of the present invention to provide compositions for supplementation of a monogastric animal diet increasing nutrient exploitation and reducing feed conversion rate composed of ingredients which are readily available, can be provided at low cost, and are not toxic. This technical problem has been solved by compositions as claimed by the present invention.

It is one further object of the present invention to provide feed additives comprising said compositions.

It is another object of the present invention to provide methods of using feed additives comprising said compositions.

These and further objects are met with methods and means according to the independent claims of the present invention. The dependent claims are related to specific embodiments.

## Summary of the Invention

The present invention provides compositions comprising one or more rhamnolipids for supplementation of a monogastric animal diet. The invention further relates to animal feed or feed additive comprising said composition, and to the use of said composition as a digestibility enhancer and/or absorption enhancer in monogastric animal feed.

The invention and general advantages of its features will be discussed in detail below.

## Description of the Figures

Fig. 1 shows the structure of rhamnolipids: RL1 (mono-rhamno-di-lipidic congener), RL2 (mono-rhamno-mono-lipidic congener), RL3 (di-rhamno-di-lipidic congener) and RL4 (di-rhamno-mono-lipidicongener) (Ali *et al.,* 2017)

Fig. 2 illustrates the effects of cumulative body weight gain and treatments from day 01 to day 14 of age (starter period)

Fig. 3 illustrates the effects of cumulative feed conversion ratio and treatments from day 01 to day 14 of age (starter period)

Fig. 4 illustrates the effects of cumulative body weight gain and treatments from day 15 to day 42 of age (grower period)

Fig. 5 illustrates the effects of cumulative feed conversion ratio and treatments from day 15 to day 42 of age (grower period)

Fig. 6 illustrates the effects of cumulative body weight gain and treatments from day 01 to day 42 of age (overall period)

Fig. 7 illustrates the effects of cumulative feed conversion ratio and treatments from day 01 to day 42 of age (overall period)

Fig. 8 illustrates the effects of apparent ileal digestibility of crude protein and treatments at day 42 of age

Fig. 9 illustrates the effects of apparent ileal digestibility of crude fat and treatments at day 42 of age

Fig. 10 illustrates the effects of apparent ileal digestibility of ash and treatments at day 42 of age

Fig. 11 illustrates the effects of apparent ileal digestibility of calcium and treatments at day 42 of age

Fig. 12 illustrates the effects of apparent ileal digestibility of phosphorus and treatments at day 42 of age

## Detailed Description of the Invention

Before the invention is described in detail, it is to be understood that this invention is not limited to the particular component parts of the means described or process steps of the methods described as such means and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise.

It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that this does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.

According to a first aspect, the present invention relates to a composition for supplementation of a monogastric animal diet, comprising one or more rhamnolipids. Said composition may additionally comprise amino acids, trace elements, antioxidants, enzymes, and/or vitamins.

As understood herein, a monogastric animal is an animal having a simple single-chambered stomach, in contrast to a ruminant animal, like a cow, goat, or sheep, which has a four-chambered complex stomach. Examples of monogastric animals include omnivores such as humans, rats, dogs and pigs, carnivores such as cats, birds such as poultry, including but not limited to broilers, layers, Turkeys and ducks, and herbivores such as horses and rabbits. Herbivores with monogastric digestion can digest cellulose in their diets by way of symbiotic gut bacteria. However, their ability to extract energy from cellulose digestion is less efficient than in ruminants.

A monogastric digestive system works as soon as the food enters the mouth. Saliva moistens the food and begins the digestive process. After being swallowed, the food passes from the esophagus into the stomach, where stomach acid and enzymes help to break down the food. Bile salts are stored in the gall bladder and secreted once the contents of the stomach have reached the small intestines where most fats are broken down. The pancreas secretes enzymes and alkali to neutralize the stomach acid.

The inventors have realized and demonstrated by experimental evidence that compositions comprising one or more rhamnolipids, when added for supplementation to a monogastric animal diet, can compensate for the less efficient digestion of cellulose and other fibre molecules, and hence reduced ability to extract energy and yield metabolic intermediates for anabolic metabolism, as compared to ruminants, by way of increasing ileal digestibility and body weight gain as well as decreasing feed conversion ratio.

According to one embodiment, the composition according to the present invention comprises at least one rhamnolipid, wherein from said rhamnolipid(s) at least one is selected from the group consisting of mono-rhamnolipids and di-rhamnolipids.

According to one embodiment of the present invention, in said composition the weight ratio of mono-rhamnolipids and di-rhamnolipids is in the range of 9:1 to 0.5:5, preferably in the range of 9:1 to 1:1.

Furthermore, in said composition at least one rhamnolipid can comprise one or two β-hydroxy fatty acid units. In a preferred embodiment, said β-hydroxy fatty acid units have the same chain lengths. At least one of the β-hydroxy fatty acid units of the rhamnolipid comprised in said composition is based on a fatty acid chain length of 8 to 16 carbon atoms, preferably 8 to 12 carbon atoms. In addition, said at least one β-hydroxy fatty acid unit may contain no, one or two double bonds, in particular no double bonds.

According to one embodiment of the present invention, at least one of the β-hydroxy fatty acid units of the at least one rhamnolipid comprised in said composition is β-hydroxydecanoic acid.

According to one aspect of the present invention, the at least one rhamnolipid comprised in said composition can be produced by at least one producing organism selected from the group comprising *Pseudomonas spec.*, *Pseudomonas aeruginosa*, *Acinetobacter calco-aceticus*, *Pseudoxanthomonas sp.*, *Enterobacter sp.*, *Pantoea sp.*, *Burkhoderia sp.*, *Myxococcus sp.*, *Renibacterium salmoninarum*, *Cellulomonas cellulans*, *Nocardioids sp.*, and/or *Tetragenococcus koreensis*.

In a preferred embodiment of the composition according to the present invention, the at least one rhamnolipid is a rhamnolipid that can be or has been produced by the genus *Pseudomonas*, in particular by *Pseudomonas aeruginosa*.

According to another aspect of the present invention, the composition comprising at least one rhamnolipid furthermore comprises as additional ingredient at least one selected from the group consisting of alginate and pyoverdine. Rhamnolipid compositions, including mono rhamnolipids and/or di-rhamnolipids, optionally comprising pyoverdine and/or alginate, have been disclosed in EP 3301196 A1 and WO 2018060517 A1, the content of which is incorporated by reference herein.

According to one embodiment, the at least one rhamnolipid comprised in the composition of the present invention is present in a supramolecular complex. Such complexes with, e.g., alginate and, optionally, pyoverdine, have been disclosed in EP 3301196 A1 and WO 2018060517 A1, the content of which is incorporated by reference herein.

According to a second aspect, the present invention furthermore relates to an animal feed or feed additive comprising the composition with one or more rhamnolipids as defined above. In a preferred embodiment, said animal feed or feed additive can be pre-mixed.

As understood herein, feed additives are products used in animal nutrition for purposes of improving the quality of feed and the quality of food from animal origin, or to improve the animals' performance and health, e.g. providing enhanced digestibility of the feed materials. Feed additives may not be put on the market unless authorisation has been given following a scientific evaluation demonstrating that the additive has no harmful effects on human and animal health and on the environment.

Preferably, the composition for supplementation of a monogastric animal diet, comprising one or more rhamnolipids, and/or said animal feed or feed additive is/are for at least one selected from the group consisting of monogastric species like poultry, pig, fish, and/or for companion animals and aquaculture.

According to a third aspect, the present invention furthermore relates to a method of feeding monogastric animals, comprising at least one step of supplementing the monogastric animal diet by a composition according to the present invention.

According to a fourth aspect, the present invention relates to the use of the composition as defined above as a digestibility enhancer and/or absorption enhancer in monogastric animal feed. Said use can relate to said composition improving digestibility of proteins, fats, ash and/or trace elements and/or improving energy and amino acid availability, wherein said trace elements are preferably selected from the group consisting of phosphorus and calcium.

As understood herein, the term "ash" relates to the burning residue of organic material, *i.e.,* material from plants, animals or microorganisms, or from synthetic organic material; the term chemically relates to the mineral content, *i.e.,* the inorganic moiety of the chemical compositions, elements and mixtures plants, animals, microorganisms or parts thereof are composed of. Ash predominantly consists of oxides and carbonates of various metals, e.g., $Al_2O_3$, $CaO$, $Fe_2O_3$, $MgO$, $MnO$, $P_2O_5$, $K_2O$, $SiO_2$, $Na_2CO_3$, $NaHCO_3$, etc.

In preferred embodiments of the present invention, said digestibility enhancer increases the animal body weight gain and/or feed intake and/or decreases the feed conversion ratio.

Furthermore, said digestibility enhancer can also increase enzymatic activity. In particularly preferred embodiments, said digestibility enhancer increases enzymatic activity of phytase and/or xylanase in the animal feed.

Furthermore, rhamnolipids with their ability to improve digestibility and absorption of nutrients in the gastrointestinal tract of monogastric animals can improve the uptake of enzymatic degradation products and improve performance of exogenous enzymes added in animal feeds (*e.g.,* phytase, xylanase and proteases). The improvement in fat digestibility by rhamnolipids in the diet, as demonstrated by the inventors, further indicates that the digestibility enhancer will assist uptake of certain fat soluble micronutrients such as vitamins in the gastrointestinal tract and contribute to growth and overall wellbeing of the animal.

In preferred embodiments of the present invention, said use of the composition relates to the use for a monogastric animal, wherein said animal is selected from the group consisting of poultry, preferably chicken, turkey, duck, pig, fish, shrimp, dog, cat, horse, pre-ruminant calf.

Grown cattle have a stomach divided into four working parts: the rumen (paunch), the reticulum (honeycomb), the omasum (bible), and the abomasum (true stomach). The rumen is very large compared with the other sections. In the very young calf (birth to ca. 3 weeks), the abomasum is the major functional compartment, making up ca. 80% of the total stomach. The other compartments grow quickly once the calf begins eating dry feeds. As used herein, the term "pre-ruminant calf" relates to a calf at the time taken by the calf to change from using just the abomasum to efficiently using all four stomachs, *i.e.,* before the rumen develops.

For use of the composition according to the present invention, said composition is dosed at 10 mg/kg feed to 710 mg/kg feed, preferably at 50 mg/kg feed to 710 mg/kg feed, in the animal feed.

## Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

In order to evaluate the efficacy of a Digestibility Enhancer compound comprising rhamnolipid ("JRV-L-V3 750") in a livestock of non-ruminants, said compound was added in form of a premix to basal starter and grower diets for male broiler chickens over a 42-day feeding period at two different dose levels. For comparison, broiler chickens were fed the corresponding basal diets without Digestibility Enhancer supplementation but with addition of limestone used as vehicle in the tested Digestibility Enhancer premix (placebo). In accordance to the guidelines given by EFSA (The EFSA Journal (2012), 10 (1):2536) the effects of the supplementation were demonstrated on performance (body weight, body weight gain, feed intake, feed conversion ratio) over the feeding period, and on apparent ileal digestibility (crude protein, crude fat, ash, calcium, phosphorus) at the end of the feeding period.

**Example 1: Study Design and Experimental Procedures**

1. Digestibility Enhancer

For production of the Digestibility Enhancer compound (JRV-L-V3 750), first a *Pseudomonas aeruginosa* strain was cultivated in liquid media. The inoculum was transferred into a bioreactor for fermentation processes under controlled conditions (pressure, time, temperature). After a defined fermentation time, post-fermentation processes occurred, like precipitation and concentration of the product. After drying and subsequent lipid extraction, the solid product had a rhamnolipid composition as follows:

Di-Rhamnolipids: 65-70%
Mono-Rhamnolipids: 15-20%.

2. Treatment groups and Conditions

The study was performed using 90 one-day-old healthy male broiler chickens (Cobb 500) which were allocated at random to 30 identical stainless-steel cages with three birds per cage (10 repetitions per any treatment group). Environment and management were as far as possible adjusted to standard conditions used in commercial broiler farms. The overall bird losses amounted to 4.4%, which suggested the absence of sub-clinical problems in the flock and, therefore, an optimal background of health condition. Medical treatments were not necessary.

Efficacy of the Digestibility Enhancer compound "JRV-L-V3 750" supplemented into basal starter and grower diets fed to broiler chickens from day 01 to day 42 of age (42 d feeding period) at a lower or tenfold of the lower dose level (starter diet: 46.8 mg/kg or 468 mg/kg; grower diet: 70.6 mg/kg or 706 mg/kg). For comparison, broiler chickens were fed similar basal diets without Digestibility Enhancer supplementation but with addition of limestone used as vehicle in the tested Digestibility Enhancer premix (placebo). The effects were demonstrated on performance (body weight, body weight gain, feed intake, feed conversion ratio) from day 01 to day 42 of age and on apparent ileal digestibility (crude protein, crude fat, ash, calcium, phosphorus) at the end of the 42-day feeding period (day 42 of age).

The design of the efficacy test included three treatment groups. The first treatment group (T1) was the placebo group fed basal diets without Digestibility Enhancer addition but with using limestone as vehicle in the tested Digestibility Enhancer premix. Treatment diets T2 and T3 were similar to the placebo group but supplemented with the Digestibility Enhancer compound "JRV-L-V3 750" at the lower or at the tenfold dose level. Details are given in Table 1.

**Table 1:** Overview of the treatments applied to male broiler chickens from day 01 to day 42 of age

| Treatment groups | | T1 | T2 | T3 |
|---|---|---|---|---|
| Broiler chickens | n° | 30 | 30 | 30 |
| Replicates | n° | 10 | 10 | 10 |
| JRV-L-V3 750 | | | | |
| - Starter diet | mg/kg | | 46.8 | 468 |
| - Grower diet | mg/kg | | 70.6 | 706 |

During the 42-day feeding period, temperature, relative humidity, lighting and forced ventilation (air speeds from 0.3 (1 to 18 days of age) up to 0.6 m/s from 19 days of age onwards) was controlled throughout the 42-day feeding period. In case of anomalies, parameters were automatically adjusted to the targets. Room temperature was gradually reduced from 33°C at day 1 of age to about 23°C from day 28 of age onwards. The relative humidity was in the range of 50 to 60%. The artificial light (45 lux) during the first 4 days of age was provided for 24 h continuously. From day 5 of age onwards, the lighting regime consisted in an 18h light (about 45 lux) and 6h dark cycle. Broiler chickens had *ad libitum* access to feed (mash form) and water throughout the 42-day feeding period; the water supply was equipped with nipples.

3. Experimental Diets and Feeding System

The 42-day feeding period was divided in two feeding phases: a starter period from day 01 to day 14 of age followed by a grower period from day 15 to day 42 of age, respectively. The basal starter and grower diet was calculated to meet or slightly exceed the nutrient requirements for broiler chickens recommended by the Society of Nutrition Physiology (1999). Diets were manufactured in mash form and prepared without inclusion of enzymes, growth promoters, acidifiers, probiotics or coccidiostats. The feed ingredients used and the nutritional composition of the experimental diets are shown in Tables 2 and 3.

**Table 2:** Ingredients and nutritional composition (as fed) of the starter diets (day 01 to day 14 of age)

| Treatment groups | | T1 | T2 | T3 |
|---|---|---|---|---|
| | | Ingredients | | |
| Maize | % | 33.24 | 33.24 | 33.24 |
| Soybean meal (CP 44%) | % | 32.00 | 32.00 | 32.00 |
| Wheat | % | 25.09 | 25.09 | 25.09 |
| Soybean oil | % | 5.08 | 5.08 | 5.08 |
| Limestone | % | 1.5800 | 1.5753 | 1.5332 |
| Monocalcium-phosphate | % | 1.40 | 1.40 | 1.40 |
| Premix[1] | % | 1.20 | 1.20 | 1.20 |
| DL-Methionine | % | 0.26 | 0.26 | 0.26 |
| L-Lysine HCL | % | 0.13 | 0.13 | 0.13 |
| L-Threonine | % | 0.02 | 0.02 | 0.02 |
| JRV-L-V3 750 (Table 1) | % | | 0.0047 | 0.0468 |
| | | Nutritional composition | | |
| $ME_N$[2] | MJ/kg | 12.48 | 12.48 | 12.48 |
| Crude protein | g/kg | 220.80 | 220.80 | 220.80 |
| Lysine | g/kg | 12.70 | 12.70 | 12.70 |
| Methionine | g/kg | 5.90 | 5.90 | 5.90 |
| Methionine/Cystine | g/kg | 9.70 | 9.70 | 9.70 |
| Tryptophan | g/kg | 2.50 | 2.50 | 2.50 |
| Threonine | g/kg | 8.60 | 8.60 | 8.60 |
| Crude fibre | g/kg | 24.50 | 24.50 | 24.50 |
| Crude fat | g/kg | 73.60 | 73.60 | 73.60 |
| Starch | g/kg | 360.60 | 360.60 | 360.60 |
| Sugars | g/kg | 42.90 | 42.90 | 42.90 |
| Calcium | g/kg | 9.00 | 9.00 | 9.00 |
| Total phosphorus | g/kg | 7.00 | 7.00 | 7.00 |
| Sodium | g/kg | 1.60 | 1.60 | 1.60 |

[1] Contents per kg premix: 400000 IU vit. A; 40000 IU vit. D3; 8000 mg vit. E ($\alpha$-tocopherole acetate); 300 mg vit. K3; 250 mg vit. B1; 250 mg vit. B2; 2500 mg nicotinic acid; 400 mg vit. B6; 2000 $\mu$g vit. B12; 25000 $\mu$g biotin; 1000 mg calcium pantothenic acid; 100 mg folic acid; 80000 mg choline chloride; 5000 mg Zn (zinc oxide); 2000 mg Fe (iron carbonate); 6000 mg Mn (manganese oxide); 1200 mg Cu (copper sulfate-pentahydrate); 45 mg J (calcium jodate; 30 mg Co (cobalt- (II)-sulfate-heptahydrate); 35 mg Se (sodium selenite); 130 g Na (sodium chloride); 55 g Mg (magnesium oxide);
[2] Estimated according to equation of WPSA 1984.

**Table 3:** Ingredients and nutritional composition (as fed) of the grower diets (day 15 to day 42 of age)

| Treatment groups | | T1 | T2 | T3 |
|---|---|---|---|---|
| | | Ingredients | | |
| Maize | % | 33.23 | 33.23 | 33.23 |

(continued)

| Treatment groups | | T1 | T2 | T3 |
|---|---|---|---|---|
| | | Ingredients | | |
| Wheat | % | 30.16 | 30.16 | 30.16 |
| Soybean meal (CP 44%) | % | 26.50 | 26.50 | 26.50 |
| Soybean oil | % | 5.08 | 5.08 | 5.08 |
| Limestone | % | 1.5600 | 1.5529 | 1.4894 |
| Monocalcium phosphate | % | 1.38 | 1.38 | 1.38 |
| Premix [1] | % | 1.20 | 1.20 | 1.20 |
| Titanium(IV) dioxide | % | 0.40 | 0.40 | 0.40 |
| L-Lysine HCL | % | 0.23 | 0.23 | 0.23 |
| DL-Methionine | % | 0.20 | 0.20 | 0.20 |
| Threonine | % | 0.06 | 0.06 | 0.06 |
| JRV-L-V3 750 (Table 1) | % | | 0.0071 | 0.0706 |
| | | Nutritional composition | | |
| $ME_N$ [2] | MJ/kg | 12.56 | 12.56 | 12.56 |
| Crude protein | g/kg | 200.80 | 200.80 | 200.80 |
| Lysine | g/kg | 12.00 | 12.00 | 12.00 |
| Methionine | g/kg | 5.00 | 5.00 | 5.00 |
| Methionine/Cystine | g/kg | 8.50 | 8.50 | 8.50 |
| Tryptophan | g/kg | 2.30 | 2.30 | 2.30 |
| Threonine | g/kg | 8.10 | 8.10 | 8.10 |
| Crude fibre | g/kg | 24.00 | 24.00 | 24.00 |
| Crude fat | g/kg | 73.90 | 73.90 | 73.90 |
| Starch | g/kg | 390.80 | 390.80 | 390.80 |
| Sugars | g/kg | 39.50 | 39.50 | 39.50 |
| Calcium | g/kg | 8.80 | 8.80 | 8.80 |
| Total phosphorus | g/kg | 6.70 | 6.70 | 6.70 |
| Sodium | g/kg | 1.70 | 1.70 | 1.70 |

[1] Contents per kg Premix: 400000 I.U. vit. A (acetate); 120000 I.U. vit. D3; 8000 mg vit. E ($\alpha$-tocopherole acetate); 200 mg vit. K3 (MSB); 250 mg vit. B1 (mononitrate); 420 mg vit. B2 (cryst. riboflavin); 2500 mg niacin (niacinamide); 400 mg Vit. B6 (HCl); 2000 $\mu$g vit. B12; 25000 $\mu$g Biotin (commercial, feed grade); 1000 mg pantothenic acid (Ca d-pantothenate); 100 mg folic acid (cryst. commercial feed grade); 80000 mg choline (chloride); 5000 mg Zn (sulfate); 5000 mg Fe (carbonate); 6000 mg Mn (sulfate); 1000 mg Cu (sulfate-pentahydrate); 20 mg Se (Na-selenite); 45 mg J (Ca-iodate); 130 g Na (NaCl); 55 g Mg (sulfate);
[2] Estimated according to equation of WPSA 1984.

4. Performance Parameters

On the initial day of the experiment and at the end of each following week performance (body weight, body weight gain, feed intake, feed conversion ratio) was recorded per pen (replicate unit) from day 01 to day 42 of age. The operations were done at the same time, in the same cage order and with the same personnel.

For calculation of the **body weight gain** per broiler chicken the following formula was used:

$$\text{Average weight gain per bird for each period} =$$

$$F - S \text{ (corrected by weight gain of died or culled chickens)}$$

F - Average weight of the live birds in the cage at the weighing day
S - Average weight of the live birds in the cage at the previous weighing

The **feed intake** (corrected for dispersed feed) was calculated as follows:

$$\text{Feed intake per period} = \frac{\text{Total feed consumed per cage}}{(\text{Number of surviving birds x days of the period}) + \text{days of died birds alive}}$$

The **feed conversion ratio** was based on the following formula:

$$\text{Feed conversion per period} = \frac{\text{Total feed consumed for the period in each replicate}}{\text{Total weight gain for the period (with weight gain of died or culled chickens)}}$$

**Apparent ileal digestibility** was determined in all birds of each treatment group at the end of the 42-day feeding period (day 42 of age). For this purpose, the birds were killed by decapitation after stunning of the animals about 5 hours after starting the lighting cycle. The posterior half between Meckels' diverticulum and 3 cm cranial to the *osteum ileocaecale* was restricted. Ileal digesta was collected by purging the ileum with a defined amount (5 ml) of water. The ileal digesta of 3 birds per each cage was pooled. Before analyzing the samples were stored at -20°C before being freeze-dried for chemical analyses. Titanium(IV) oxide ($TiO_2$) was supplemented as an inert marker at the dose level of 4 g/kg diet. For calculation of the apparent ileal digestibility the following formula was used:

$$\text{Apparent Ileal Digestibility (\%)} = 100 - \left[ \frac{\text{\% Marker in feed}}{\text{\% Marker in ileum}} \times \frac{\text{\% Nutrient in ileum}}{\text{\% Nutrient in feed}} \right] \times 100$$

5. Analytical Methods and Statistical Analysis

All experimental diets were ground to pass through a 0.25-mm screen before analysis. Laboratory measurements were including Weender constituents and additionally starch, total sugars, calcium, phosphorus and sodium. Analyses were in accordance to the methods issued by VDLUFA (dry matter: VDLUFA III 3.1; crude protein: VDLUFA III 4.1.1 modified according to macro-N determination (vario Max CN); crude fibre: VDLUFA III 6.1.4; crude ash: VDLUFA III 8.1; crude fat: VDLUFA III 5.1.1; starch: VDLUFA III 7.2.1; total sugars: VDLUFAIII 7.1.1; calcium: VDLUFA VII 2.2.2.6; phosphorus: VDLUFA VII 2.2.2.6; sodium: VDLUFA VII2.2.2.6).

Furthermore, freeze-dried ileal digesta pooled per cage was ground to pass a 0.25-mm screen and analyzed for dry matter, crude protein, crude fat, crude ash, calcium and phosphorus (dry matter:VDLUFAIII3.1; crude protein: VDLUFAIII4.1.1 modified according to macro-N determination (vario Max CN); crude fat: VDLUFA III 5.1.1; crude ash: VDLUFAIII 8.1; calcium: VDLUFA VII 2.2.2.6; phosphorus: VDLUFA VII2.2.2.6). Titanium(IV) dioxide content in feed and ileal digesta was measured using a UV spectrophotometer following the method of Myers *et al.* (2004).

Results are presented as means ± standard deviation. The statistical model was including the random effects of animal and the fixed effect of treatment. The statistical analyses were performed with the software package SPSS (IBM SPSS Version 21) and based on one way ANOVA. All treatment least squares means were compared with each other and the Tukey adjustment was used to control for experiment related (ß) error. Differences among least squares means with a probability of $P < 0.05$ were accepted as statistically significant, also mean differences with P-values ranging from 0.06 to 0.10 were accepted as trends.

**Example 2: Study Results**

The overall body weight gain and overall feed conversion ratio of broiler chickens fed diets without addition of "JRV-L-V3 750" reached 2808 g (66.8 g per day) and 1.517, respectively. When feeding broiler chickens diets with inclusion of "JRV-L-V3 750" the overall body weight gain was significantly increased with dose and was up to 5.3% higher than that recorded for broiler chickens fed diets without Digestibility Enhancer supplementation. This was associated with a dose-dependent statistical relevant decrease of the overall feed conversion ratio up to -3.8% when compared to broiler chickens fed diets without using Digestibility Enhancer addition.

Moreover, treatment effects on performance seemed to be greater during the grower period which was obviously due to the higher dietary Digestibility Enhancer concentration in the grower diets when compared to those added into the starter diets (+50.9%).

Statistical relevant beneficial effects of "JRV-L-V3 750" on performance were combined with the significantly improved apparent digestibility in broiler chickens fed diets at the recommended or at the tenfold of the recommended dose level when compared to broiler chickens fed diets without Digestibility Enhancer addition. Broiler chickens fed diets containing "JRV-L-V3 750" at the tenfold of the recommended dose level had the most prominent response. Differences between both dose levels were significant with regard to crude protein and calcium.

The results of the present study concerning the efficacy of "JRV-L-V3 750" supplemented at a lower or at a tenfold dose level into corn-wheat-soybean meal diets could demonstrate statistical relevant benefits on performance and apparent ileal digestibility in comparison to broiler chickens fed similar basal diets without Digestibility Enhancer addition. A distinct plateau could not be achieved within the level of supplementation studied. These findings suggest that feeding diets supplemented with "JRV-L-V3 750" already at the lower dose level could provide statistical relevant benefits on performance and ileal apparent digestibility.

In detail, the effects of adding the Digestibility Enhancer compound "JRV-L-V3 750" to diets for broiler chickens on the performance parameters measured from day 01 to day 42 of age are presented either weekly or cumulative in Tables 4 and 5, respectively.

1. Body Weight and Body Weight Gain

The body weight of broiler chickens at the start of the experiment was at around 41.6 g and as a matter of the selection nearly similar in all treatment groups. The cumulative body weight gain (day 01 to day 42 of age) of broiler chickens fed diets without addition of "JRV-L-V3 750" (T1) reached 2808 g (66.8 g per day) and was within the targets given by the breeder (2797 g). When feeding broiler chickens diets with inclusion of "JRV-L-V3 750" at the lower (T2) or tenfold dose level (T3), the cumulative body weight gain increased significantly up to 5.3% (T3). Differences were statistically significant when feeding diets containing the lower dose level, and results between the lower and the tenfold dose level were significant. The beneficial effects of "JRV-L-V3 750" on overall body weight gain were mainly due to the positive response during the grower period.

The overall feed intake of broiler chickens fed diets without using "JRV-L-V3 750" amounted to 4259 g (101.4 g per day). Results for broiler chickens fed diets containing "JRV-L-V3 750" were slightly higher in comparison to those fed diets without supplemented "JRV-L-V3 750" (T2: +0.5; T3: +1.3%).

**Table 4:** Effect of "JRV-L-V3 750" on performance in broiler chickens from day 01 to day 28 of age

| Treatment groups | | T1 | T2 | T3 | P value |
|---|---|---|---|---|---|
| Total birds per group | n° | 30 | 30 | 30 | |
| Repetitions | n° | 10 | 10 | 10 | |
| JRV-L-V3 750 | | | | | |
| - Starter diet (d 01-d 14 of age) | mg/kg | | 46.8 | 468.0 | |
| - Grower diet (d 15-d 42 of age) | mg/kg | | 70.6 | 706.0 | |
| | | d 01 to d 07 of age | | | |
| Broiler chickens | n° | 29 | 29 | 30 | |
| Body weight start | g | $41.6 \pm 0.6$ | $41.5 \pm 0.8$ | $41.5 \pm 0.6$ | 0.812 |
| Body weight end | g | $146.1 \pm 8.1^a$ | $153.6 \pm 5.3^b$ | $155.2 \pm 4.4^b$ | **0.006** |
| Body weight gain | g | $104.5 \pm 8.5^a$ | $112.1 \pm 5.6^b$ | $113.8 \pm 4.5^b$ | **0.007** |
| Feed intake | g | $115.5 \pm 8.5$ | $117.3 \pm 6.1$ | $119.5 \pm 5.7$ | 0.447 |
| Feed conversion [1] | | $1.108 \pm 0.067^a$ | $1.047 \pm 0.038^b$ | $1.051 \pm 0.044^{ab}$ | **0.023** |
| | | d 08 to d 14 of age | | | |
| Broiler chickens | n° | 29 | 29 | 29 | |
| Body weight start | g | $146.1 \pm 8.1^a$ | $153.6 \pm 5.3^b$ | $155.2 \pm 4.4^b$ | **0.006** |
| Body weight end | g | $450.3 \pm 15.7^a$ | $462.9 \pm 14.4^{ab}$ | $474.0 \pm 21.7^b$ | **0.019** |
| Body weight gain | g | $304.2 \pm 11.0$ | $309.3 \pm 13.7$ | $318.8 \pm 23.6$ | 0.166 |
| Feed intake | g | $360.1 \pm 10.5$ | $363.7 \pm 13.4$ | $373.0 \pm 18.6$ | 0.145 |
| Feed conversion [1] | | $1.186 \pm 0.061$ | $1.178 \pm 0.076$ | $1.178 \pm 0.125$ | 0.976 |

(continued)

| | | d 15 to d 21 of age | | | |
|---|---|---|---|---|---|
| Broiler chickens | n° | 28 | 29 | 29 | |
| Body weight start | g | 450.3 ± 15.7" | 462.9 ± 14,4[ab] | 474.0 ± 21.7[b] | **0.019** |
| Body weight end | g | 887.4 ± 23.1[a] | 909.9 ± 27.0[ab] | 933.2 ± 26.9[b] | **0.002** |
| Body weight gain | g | 437.2 ± 24.0 | 447.0 ± 18.1 | 459.1 ± 32.9 | 0.179 |
| Feed intake | g | 654.5 ± 36.9 | 672.9 ± 33.6 | 667.9 ± 33.0 | 0.479 |
| Feed conversion [1] | | 1.499 ± 0.072 | 1.506 ± 0.074 | 1.459 ± 0.088 | 0.357 |
| | | d 22 to d 28 of age | | | |
| Broiler chickens | n° | 28 | 29 | 29 | |
| Body weight start | g | 887.4 ± 23.1[a] | 909.9 ± 27.0[ab] | 933.2 ± 26.9[b] | **0.002** |
| Body weight end | g | 1472.2 ± 25.4[a] | 1517.5 ± 30.8[b] | 1569.1 ± 36.8[c] | **<0.001** |
| Body weight gain | g | 584.8 ± 37.6[a] | 607.6 ± 31.0[ab] | 635.9 ± 27.7[b] | **0.006** |
| Feed intake | g | 869.8 ± 31.8 | 864.7 ± 40.4 | 883.5 ± 55.4 | 0.615 |
| Feed conversion [1] | | 1.491 ± 0.070[a] | 1.424 ± 0.035[b] | 1.389 ± 0.055[b] | **0.001** |
| | | | | | |
| | | d 29 to d 35 of age | | | |
| Broiler chickens | n° | 28 | 29 | 29 | |
| Body weight start | g | 1472.2 ± 25.4[a] | 1517.5 ± 30.8[b] | 1569.1 ± 36.8[c] | **<0.001** |
| Body weight end | g | 2084.6 ± 35.2[a] | 2135.5 ± 42.8[b] | 2193.6 ± 49.8[c] | **<0.001** |
| Body weight gain | g | 612.4 ± 30.5 | 618.1 ± 26.4 | 624.5 ± 26.0 | 0.627 |
| Feed intake | g | 953.5 ± 27.5 | 946.9 ± 27.8 | 940.4 ± 25.2 | 0.560 |
| Feed conversion [1] | | 1.560 ± 0.087 | 1.535 ± 0.095 | 1.509 ± 0.095 | 0.480 |
| | | d 36 to d 42 of age | | | |
| Broiler chickens | n° | 28 | 29 | 29 | |
| Body weight start | g | 2084.6 ± 35.2[a] | 2135.5 ± 42.8[b] | 2193.6 ± 49.8[c] | **<0.001** |
| Body weight end | g | 2849.3 ± 45.3[a] | 2912.6 ± 27.8[b] | 2996.6 ± 28.5[c] | **<0.001** |
| Body weight gain | g | 764.7 ± 38.7 | 777.0 ± 54.7 | 803.0 ± 42.7 | 0.182 |
| Feed intake | g | 1305.9 ± 48.8 | 1314.4 ± 65.9 | 1331.6 ± 66.6 | 0.635 |
| Feed conversion [1] | | 1.709 ± 0.045 | 1.695 ± 0.082 | 1.659 ± 0.043 | 0.170 |
| [1] kg feed per kg body weight gain; [ab] different superscripts within lines indicate levels of significance at P < 0.05 | | | | | |

**Table 5:** Effect of "JRV-L-V3 750" on performance in broiler chickens during the starter, grower, and overall feeding period

| Treatment groups | | T1 | T2 | T3 | P value |
|---|---|---|---|---|---|
| Total birds per group | n° | 30 | 30 | 30 | |
| Repetitions | n° | 10 | 10 | 10 | |
| JRV-L-V3 750 - Starter diet (d 01 - d 14 of age) | mg/kg | | 46.8 | 468.0 | |
| - Grower diet (d 15 - d 42 of age) | mg/kg | | 70.6 | 706.0 | |
| | | Starter period (d 01 to d 14 of age) | | | |
| Broiler chickens | n° | 29 | 29 | 29 | |
| Body weight start | g | 41.6 ± 0.6 | 41.5 ± 0.8 | 41.5 ± 0.6 | 0.812 |
| Body weight end | g | 450.3 ± 15.7[a] | 462.9 ± 14.4[ab] | 474.0 ± 21.7[b] | **0.019** |

(continued)

| | | Starter period (d 01 to d 14 of age) | | | |
|---|---|---|---|---|---|
| Body weight gain | g | $408.6 \pm 16.0^a$ | $421.4 \pm 14.9^{ab}$ | $432.6 \pm 21.8^b$ | **0.020** |
| Feed intake | g | $475.6 \pm 13.9$ | $481.0 \pm 14.6$ | $492.5 \pm 18.0$ | 0.065 |
| Feed conversion [1] | | $1.165 \pm 0.043$ | $1.143 \pm 0.063$ | $1.142 \pm 0.086$ | 0.685 |
| | | Grower period (d 15 to d 42 of age) | | | |
| Broiler chickens | n° | 28 | 29 | 29 | |
| Body weight start | g | $450.3 \pm 15.7^a$ | $462.9 \pm 14.4^{ab}$ | $474.0 + 21.7^b$ | **0.019** |
| Body weight end | g | $2849.3 \pm 45.3^a$ | $2912.6 \pm 27.8^b$ | $2996.6 \pm 28.5^c$ | **<0.001** |
| Body weight gain | g | $2399.0 \pm 57.7^a$ | $2449.6 \pm 28.2^b$ | $2522.5 \pm 36.0^c$ | **<0.001** |
| Feed intake | g | $3783.7 \pm 75.2$ | $3798.7 \pm 59.0$ | $3823.4 \pm 54.2$ | 0.383 |
| Feed conversion [1] | | $1.578 \pm 0.028^a$ | $1.551 \pm 0.025^b$ | $1.516 \pm 0.016^c$ | **<0.001** |
| | | Overall period (d 01 to d 42 of age | | | |
| Broiler chickens | n° | 28 | 29 | 29 | |
| Body weight start | g | $41.6 \pm 0.6$ | $41.5 \pm 0.8$ | $41.5 \pm 0.6$ | 0.812 |
| Body weight end | g | $2849.3 \pm 45.3^a$ | $2912.6 \pm 27.8^b$ | $2996.6 \pm 28.5^c$ | **<0.001** |
| Body weight gain | g | $2807.6 \pm 45.0^a$ | $2871.1 \pm 27.8^b$ | $2955.1 \pm 28.3^c$ | **<0.001** |
| Feed intake | g | $4259.4 \pm 70.6$ | $4279.8 \pm 53.5$ | $4315.8 \pm 63.0$ | 0.145 |
| Feed conversion [1] | | $1.517 \pm 0.020^a$ | $1.491 \pm 0.021^b$ | $1.460 \pm 0.013^c$ | **<0.001** |
| [1] kg feed per kg body weight gain; [ab] different superscripts within lines indicate levels of significance at $P < 0.05$ | | | | | |

## 2. Feed Conversion Ratio

The overall feed conversion ratio (kg feed per kg body weight gain) of broiler chickens fed the basal diets without Digestibility Enhancer addition amounted to 1.517, and was about - 11.2% lower than the targets given by the breeder (1.700). Broiler chickens fed diets with supplementation of "JRV-L-V3 750" had a significantly dose dependent reduced overall feed conversion ratio in comparison to that shown for broiler chickens fed diets without Digestibility Enhancer addition (T2: -1.7%, T3: -3.8%). In accordance to overall body weight gain results between birds fed diets at the lower or at the tenfold dose level were significant.

## 3. Apparent Ileal Digestibility

At the end of the 42-day feeding period all birds per treatment were killed after stunning about five hours after starting the lighting cycle for measurements of apparent ileal digestibility. Results are presented in Table 6. For assuring sufficient ileal digesta always two or three birds per cage (one repetition per treatment) were pooled.

**Table 6:** Effect of "JRV-L-V3 750" on apparent ileal digestibility at the end of the 42-day feeding period (day 42 of age)

| Treatment groups | | T1 | T2 | T3 | P value |
|---|---|---|---|---|---|
| Broiler chickens | n° | 30 | 30 | 30 | |
| Replicates | n° | 10 | 10 | 10 | |
| JRV-L-V3 750 - Starter diet (d 01 - d 14 of age) | mg/kg | | 46.8 | 468.0 | |
| - Grower diet (d 15 - d 42 of age) | mg/kg | | 70.6 | 706.0 | |
| | | Body weight (d 42 of age) | | | |
| Body weight | g | $2849.3 \pm 45.3^a$ | $2912.6 \pm 27.8^b$ | $2996.6 \pm 28.5^c$ | **<0.001** |

(continued)

| | | Dry matter of Ileal digesta (d 42 of age) | | | |
|---|---|---|---|---|---|
| Dry matter | % | 42.96 ± 1.46 | 42.77 ± 1.61 | 42.12 ± 1.94 | 0.507 |
| | | Apparent ileal digestibility (d 42 of age) | | | |
| Crude protein | % | 78.96 ± 0.59[a] | 80.67 ± 0.75[b] | 81.96 ± 1.42[c] | **<0.001** |
| Crude fat | % | 89.27 ± 1.89[a] | 93.30 ± 0.48[b] | 94.44 ± 0.51[b] | **<0.001** |
| Ash | % | 38.58 ± 1.74[a] | 40.87 ± 1.52[b] | 41.61 ± 1.62[b] | **0.001** |
| Calcium | % | 34.16 ± 1.08[a] | 35.46 ± 0.73[b] | 36.74 ± 1.05[c] | **<0.001** |
| Phosphorus | % | 39.60 ± 0.93[a] | 41.25 ± 1.70[b] | 42.13 ± 0.97[b] | **<0.001** |
| [ab] Means with different superscripts within the row differ significantly (P<0.05). | | | | | |

The dry matter of the pooled ileum digesta per pen reached on average 42.6%. With increasing dose levels the dry matter content was slightly decreased up to approximately -2% when compared to broiler chickens fed diets without containing "JRV-L-V3 750". The apparent ileal digestibility of selected nutrients (ash, crude protein, crude fat, calcium, phosphorus) determined in broiler chickens fed diets without Digestibility enhancer was within the expected range. The incorporation of the Digestibility Enhancer compound "JRV-L-V3 750" at the lower dose level attributed a significant increase on apparent ileal digestibility of analyzed nutrients in comparison to broiler chickens fed diets without Digestibility Enhancers (crude protein: +2.2%; crude fat: +4.5%; ash: +5.9%; calcium: +3.8%; phosphorus: +4.2%). When feeding diets at the tenfold dose level the apparent ileal digestibility was higher than that recorded for broiler chickens fed diets at the lower dose level (crude protein: +1.6%; crude fat: +1.2%; ash: +1.8%; calcium: +3.6%; phosphorus: +2.1%).

In conclusion, the overall body weight gain and the feed conversion ratio of broiler chickens fed diets without addition of "JRV-L-V3 750" was within (body weight gain) or even better than targets given by the breeder (feed conversion ratio). Therefore, conditions for demonstrating the efficacy of the Digestibility Enhancer compound "JRV-L-V3 750" were sufficiently confirmed. When feeding broiler chickens diets with inclusion of "JRV-L-V3 750", the overall body weight gain was significantly increased with dose and was up to 5.3% higher than that recorded for broiler chickens fed diets without Digestibility Enhancer supplementation. This was associated with a dose dependent statistical relevant decrease of the overall feed conversion ratio up to -3.8% when compared to broiler chickens fed diets without Digestibility Enhancer addition.

The statistical relevant benefits on performance seemed to be due to the significantly improved apparent digestibility in comparison to broiler chickens fed diets without Digestibility Enhancer supplementation. Already at the lower dose level "JRV-L-V3 750" could provide statistical relevant improvements.

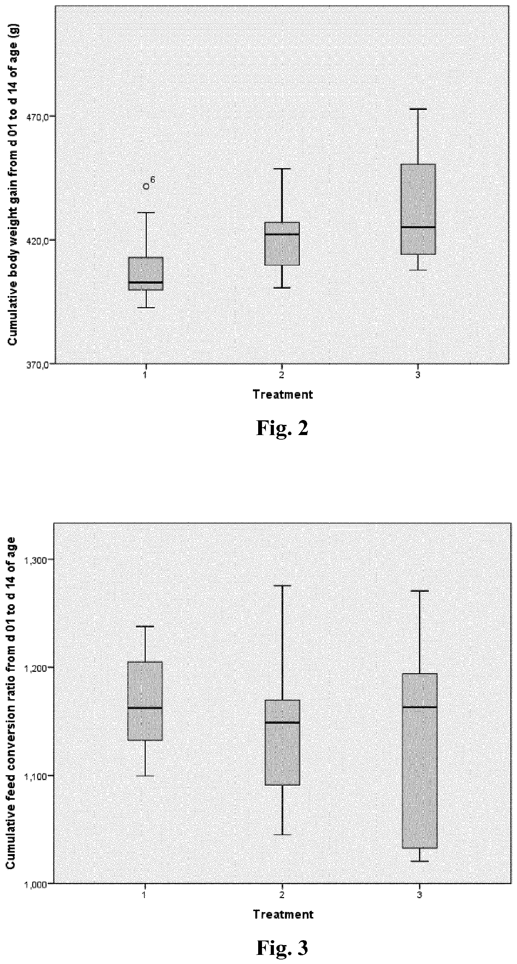

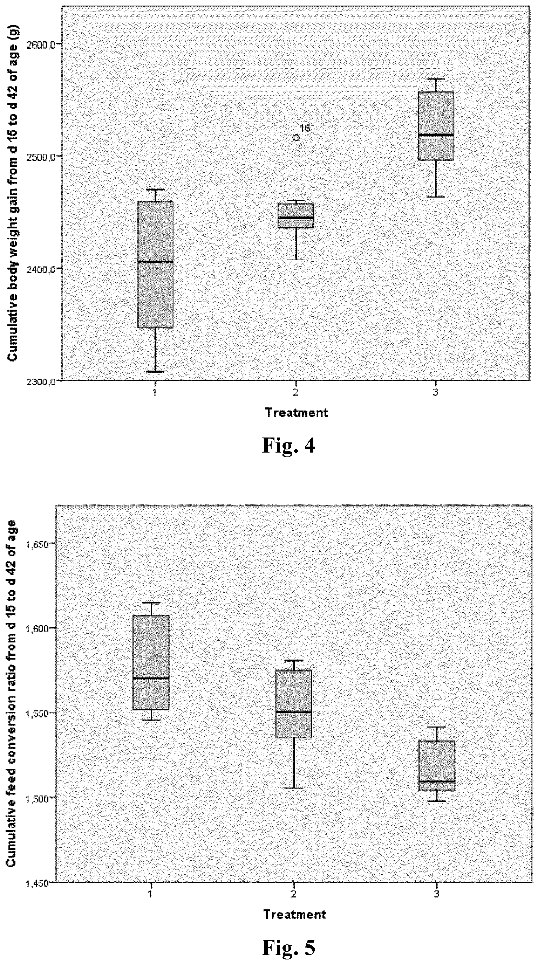

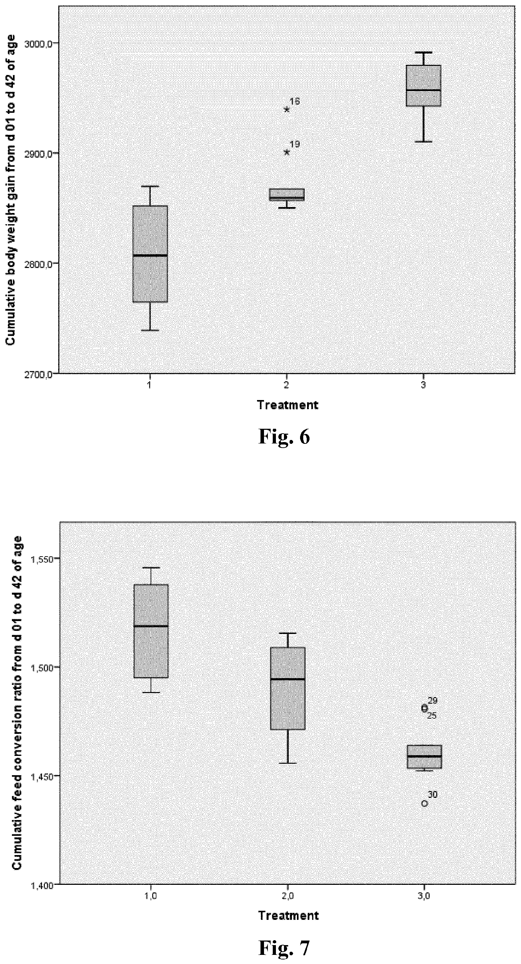

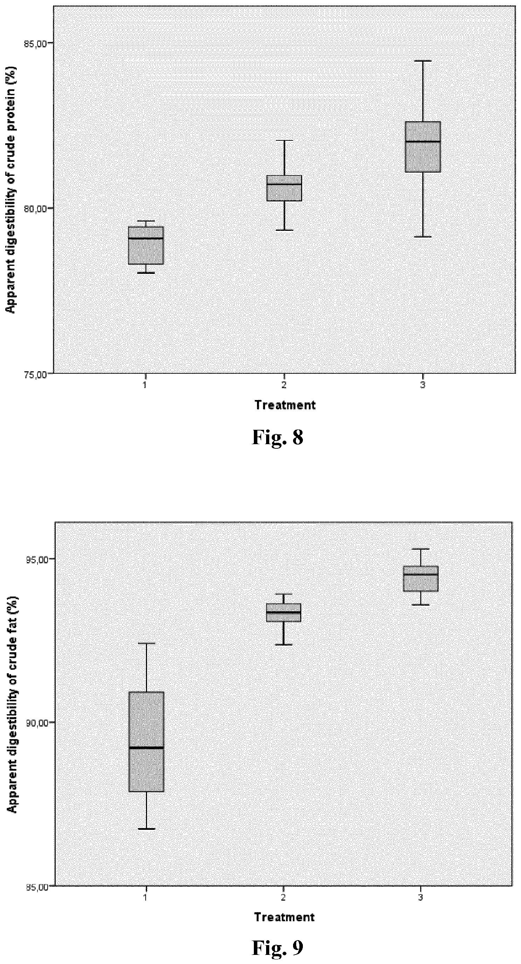

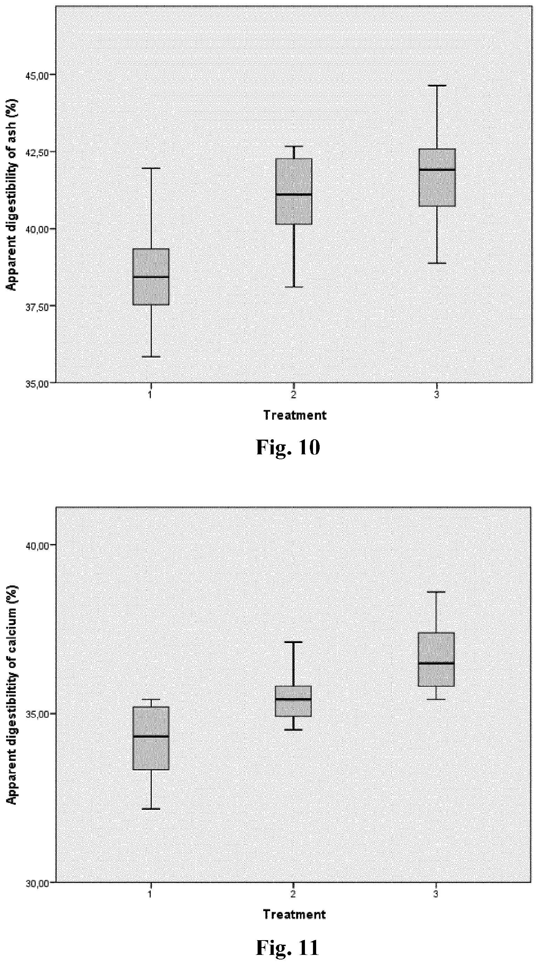

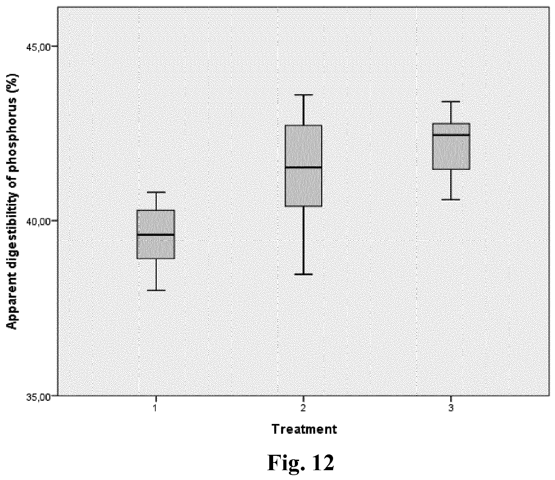

Especially for better ranking, box-and-whisker plots as exploratory graphics were additionally used to show the distribution of the dataset presented for body weight gain and feed conversion ratio as well as for apparent ileal digestibility (Figures 2 - 12). Dots represented outliers less or more than 3/2 of lower or upper quartiles.

Based on the upper and lower quartile and the lowest or greatest values excluding outliers of body weight gain, feed conversion ratio, and apparent ileal digestibility broiler chickens fed diets containing "JRV-L-V3 750" at the tenfold dose level had the most prominent response when compared to broiler chickens fed diets without "JRV-L-V3 750". Moreover, treatment related effects on performance seemed to be greater during the grower period than those noted during the starter period; that was obviously a matter of the higher dose level used in the grower diets in comparison to the starter diets.

The results of the present study showed that the efficacy of the supplemented Digestibility Enhancer product "JRV-L-V3 750" in corn-wheat-soybean meal diets could be demonstrated by statistical relevant benefits on performance, and apparent ileal digestibility at the lower or the tenfold dose level in comparison to broiler chickens fed similar basal diets without Digestibility Enhancer addition. A distinct plateau could not be achieved within the level of supplementation studied. These findings suggest that feeding diets supplemented with "JRV-L-V3 750" already at the lower dose level could provide statistical relevant benefits on performance and ileal apparent digestibility.

**References:**

Abdel-Mawgoud A.M. et al. 2010. Rhamnolipids: diversity of structures, microbial origins and roles. Appl Microbiol Biotechnol Vol. 86: 1323-1336.

Ali A.H. et al. 2017. Rhamnolipids: Preparation, Determination and Applications - A Review. American Journal of Food Science and Nutrition Research. Vol. 4, No. 1: 9-17.

Chong H. and Li Q. 2017. Microbial production of rhamnolipids: opportunities, challenges and strategies. Microb Cell Fact Vol. 16: 137-149.

Damasceno F.R.C. et al. 2012. The combined use of a biosurfactant and an enzyme preparation to treat an effluent with a high fat content. Colloids and Surfaces B: Biointerfaces. Vol. 95: 241- 246.

EFSA Panel on Additives and Products or Substances used in Animal Feed (FEEDAP); Guidance for the preparation of dossiers for zootechnical additives. 2012. EFSA Journal Vol. 10(1): 2536.

Irorere V.U. et al. 2017. Microbial rhamnolipid production: a critical re-evaluation of published data and suggested future publication criteria. Appl Microbiol Biotechnol Vol. 101: 3941-3951.

Khafagy E.S. et al. 2017. Rhamnolipids enhance in vivo oral bioavailability of poorly absorbed molecules. Pharm Res; DOI 10.1007/s11095-017-2227-y

Liu G. et al. 2018. Advances in applications of rhamnolipids biosurfactant in environmental remediation: A review. Biotechnology & Bioengineering Vol. 115 (4): 796-814.

Magalhaes L. and Nitschke M. 2013. Antimicrobial activity of rhamnolipids against Listeria monocytogenes and their synergistic interaction with nisin. Food Control Vol. 29: 138-142.

Myers W.D. et al. 2004. Technical Note: A procedure for the preparation and quantitative analysis of samples for titanium dioxide. J. Anim. Sci. Vol. 82: 179-183.

Sinumvayo J.P. and Ishimwe N. 2015. Agriculture and food applications of rhamnolipids and its production by Pseudomonas Aeruginosa. J Chem Eng Process Technol Vol. 6:2.

Society of Nutrition Physiology 1999. Empfehlungen zur Energie- und Nährstoffversorgung der Legehennen und Masthühner. Ausschuss für Bedarfsnormen der Gesellschaft für Ernährungsphysiologie. DLG-Verlags-GmbH.

Vatsa P. et al. 2010. Rhamnolipid biosurfactants as new players in animal and plant defense against microbes. Int J Mol Sci. Vol. 11: 5096-5109.

VDLUFA III 1993. The chemical analysis of feedstuffs of VDLUFA (1st - 8th supplement delivery). VDLUFA-Verlag, Speyer.

**Claims**

1. A composition for supplementation of a monogastric animal diet, comprising one or more rhamnolipids and optionally amino acids, trace elements, antioxidants, enzymes, and/or vitamins.

2. The composition according to claim 1, wherein at least one rhamnolipid is selected from the group consisting of mono-rhamnolipids and di-rhamnolipids, and/or wherein the weight ratio of mono-rhamnolipids and di-rhamnolipids is in the range of 9:1 to 0.5:5, preferably in the range of 9:1 to 1:1.

3. The composition according to any of the aforementioned claims, wherein at least one rhamnolipid comprises one or two $\beta$-hydroxy fatty acid units, preferably wherein at least one of the $\beta$-hydroxy fatty acid units is $\beta$-hydroxydecanoic acid.

4. The composition according to claim 3, wherein at least one of the $\beta$-hydroxy fatty acid units is based on a fatty acid chain length of 8 to 16 carbon atoms, preferably 8 to 12 carbon atoms.

5. The composition according to any of claims 3 or 4, wherein said at least one $\beta$-hydroxy fatty acid unit contains no, one or two double bonds, in particular no double bonds.

6. The composition according to any of the aforementioned claims, wherein the at least one rhamnolipid is a rhamnolipid that can be produced by the genus *Pseudomonas,* in particular by *Pseudomonas aeruginosa.*

7. The composition according to any of the aforementioned claims, which further comprises at least one selected from the group consisting of alginate and pyoverdine.

8. The composition according to any of the aforementioned claims, wherein the rhamnolipid is present in a supramolecular complex.

9. An animal feed or feed additive comprising the composition according to any of the aforementioned claims, optionally wherein said animal feed or feed additive can be pre-mixed.

10. Method of feeding monogastric animals, comprising a step of supplementing the monogastric animal diet by a composition according to any of claims 1 - 8 or a feed additive according to claim 9.

11. Use of the composition according to any one of claims 1 - 8 as a digestibility enhancer and/or absorption enhancer in monogastric animal feed.

12. Use of the composition according to claim 11, wherein said composition improves digestibility of proteins, fats, ash and/or trace elements and wherein said composition improves energy and/or amino acid availability, wherein said trace elements are preferably selected from the group consisting of phosphorus and calcium.

13. Use of the composition according to any of claims 11 or 12, wherein said digestibility enhancer increases the animal body weight gain and/or feed intake and/or enzymatic activity, wherein said enzymatic activity preferably is the enzymatic activity of phytase and/or xylanase in the animal feed, and/or decreases the feed conversion ratio.

14. Use of the composition according to any one of claims 11 - 13, wherein the monogastric animal is an animal selected from the group consisting of poultry, preferably chicken, turkey and duck, pig, fish, shrimp, dog, cat, horse, pre-ruminant calf.

15. Use of the composition according to any one of claims 11 - 14, wherein said composition is dosed at 10 mg/kg to 710 mg/kg, preferably at 50 mg/kg to 710 mg/kg, in the animal feed.

rhamnolipid 1, $R_1C_{10}C_{10}$

rhamnolipid 2, $R_1C_{10}$

rhamnolipid 3, $R_2C_{10}C_{10}$

rhamnolipid 4, $R_2C_{10}$

**Fig. 1**

Fig. 2

Fig. 3

Fig. 4

Fig. 5

Fig. 6

Fig. 7

**Fig. 8**

**Fig. 9**

Fig. 10

Fig. 11

**Fig. 12**

Europäisches
Patentamt

European
Patent Office

Office européen
des brevets

# EUROPEAN SEARCH REPORT

Application Number

EP 18 20 1536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 3 301 196 A1 (BIOTENSIDON INT AG [CH]) 4 April 2018 (2018-04-04)<br>* paragraph [0001]; claim 32 *<br>* paragraph [0011] *<br>* paragraph [0030] *<br>* paragraph [0012] *<br>* paragraph [0017] * | 1-10 | INV.<br>A23K10/10<br>A23K50/75<br>A23K50/30<br>A23K50/40<br>A23K50/80<br>A23K50/60 |
| X | WO 2017/220776 A1 (EW NUTRITION GMBH [DE]) 28 December 2017 (2017-12-28)<br>* page 15, paragraph 6; claim 5; figures 7-13 *<br>* page 16; table table *<br>* tables 3-4 * | 1,9-11 | ADD.<br>A61K31/7028<br>A61K31/7032 |
| X | CN 104 171 417 A (YANG HONGXING) 3 December 2014 (2014-12-03)<br>* tables 1-2 * | 1,9-11 | |
| E | WO 2018/195296 A1 (LOCUS IP COMPANY LLC [US]) 25 October 2018 (2018-10-25)<br>* the whole document * | 1,9-11 | TECHNICAL FIELDS SEARCHED (IPC) |
| A | RU 2 512 908 C1 (FEDERALNOE G BYUDZHETNOE OBRAZOVATELNOE UCHREZHDENIE VYSSHEGO PROFESSI) 10 April 2014 (2014-04-10)<br>* paragraph [0010] - paragraph [0013] * | 1-15 | A23K<br>A61K |
| A | US 2012/171350 A1 (LAI CHRON-SI [US] ET AL) 5 July 2012 (2012-07-05)<br>* paragraph [0121] - paragraph [0122] *<br>* paragraph [0121] * | 1-15 | |

-/--

The present search report has been drawn up for all claims

| Place of search | Date of completion of the search | Examiner |
|---|---|---|
| Munich | 25 January 2019 | Oenhausen, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 20 1536

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | RAJESH KUMAR ET AL: "Industrial Applications of Rhamnolipid: An Innovative Green Technology for Industry", 18 October 2018 (2018-10-18), RHAMNOLIPID BIOSURFACTANT : RECENT TRENDS IN PRODUCTION AND APPLICATION, SPRINGER, SINGAPORE, PAGE(S) 65 - 77, online, XP009510730, ISBN: 978-981-13-1288-5 [retrieved on 2018-10-18] ----- | | |

TECHNICAL FIELDS
SEARCHED        (IPC)

The present search report has been drawn up for all claims

| Place of search | Date of completion of the search | Examiner |
|---|---|---|
| Munich | 25 January 2019 | Oenhausen, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 20 1536

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2019

| Patent document cited in search report | | Publication date | Patent family member(s) | | Publication date |
|---|---|---|---|---|---|
| EP 3301196 | A1 | 04-04-2018 | EP<br>WO | 3301196 A1<br>2018060517 A1 | 04-04-2018<br>05-04-2018 |
| WO 2017220776 | A1 | 28-12-2017 | NONE | | |
| CN 104171417 | A | 03-12-2014 | NONE | | |
| WO 2018195296 | A1 | 25-10-2018 | NONE | | |
| RU 2512908 | C1 | 10-04-2014 | | | |
| US 2012171350 | A1 | 05-07-2012 | BR 112013016381 A2<br>BR 112013016478 A2<br>CA 2820960 A1<br>CA 2820965 A1<br>CA 2821319 A1<br>CA 2821770 A1<br>CA 2821935 A1<br>CN 103260440 A<br>CN 103327836 A<br>CN 103347404 A<br>CN 103384478 A<br>CN 103458708 A<br>CN 108541942 A<br>DK 2498628 T3<br>DK 2506725 T3<br>DK 2658403 T3<br>DK 2658404 T3<br>DK 2658405 T3<br>EP 2498628 A1<br>EP 2506725 A1<br>EP 2658403 A1<br>EP 2658404 A1<br>EP 2658405 A1<br>EP 3245875 A2<br>ES 2464566 T3<br>ES 2552525 T3<br>ES 2558112 T3<br>ES 2575380 T3<br>ES 2674430 T3<br>HK 1174800 A1<br>HK 1176524 A1<br>HK 1190886 A1<br>HK 1190887 A1<br>MX 343983 B<br>MX 344269 B<br>MX 344271 B | | 19-06-2018<br>20-09-2016<br>05-07-2012<br>05-07-2012<br>05-07-2012<br>05-07-2012<br>05-07-2012<br>21-08-2013<br>25-09-2013<br>09-10-2013<br>06-11-2013<br>18-12-2013<br>18-09-2018<br>23-06-2014<br>01-08-2016<br>06-06-2018<br>08-02-2016<br>11-01-2016<br>19-09-2012<br>10-10-2012<br>06-11-2013<br>06-11-2013<br>06-11-2013<br>22-11-2017<br>03-06-2014<br>30-11-2015<br>02-02-2016<br>28-06-2016<br>29-06-2018<br>10-10-2014<br>07-07-2017<br>15-07-2016<br>27-01-2017<br>01-12-2016<br>09-12-2016<br>09-12-2016 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 20 1536

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2019

| Patent document cited in search report | Publication date | Patent family member(s) | | Publication date |
|---|---|---|---|---|
| | | MY | 162776 A | 14-07-2017 |
| | | MY | 162796 A | 14-07-2017 |
| | | MY | 162797 A | 14-07-2017 |
| | | NZ | 611819 A | 30-01-2015 |
| | | NZ | 612096 A | 26-06-2015 |
| | | NZ | 612214 A | 27-02-2015 |
| | | SG | 191383 A1 | 30-08-2013 |
| | | SG | 191780 A1 | 30-08-2013 |
| | | SG | 191784 A1 | 30-08-2013 |
| | | TR | 201807445 T4 | 21-06-2018 |
| | | TW | 201306752 A | 16-02-2013 |
| | | US | 2012171350 A1 | 05-07-2012 |
| | | US | 2012172434 A1 | 05-07-2012 |
| | | US | 2012172442 A1 | 05-07-2012 |
| | | US | 2012172443 A1 | 05-07-2012 |
| | | US | 2012172445 A1 | 05-07-2012 |
| | | US | 2014249224 A1 | 04-09-2014 |
| | | US | 2015132466 A1 | 14-05-2015 |
| | | US | 2015140161 A1 | 21-05-2015 |
| | | WO | 2012092086 A1 | 05-07-2012 |
| | | WO | 2012092087 A1 | 05-07-2012 |
| | | WO | 2012092088 A1 | 05-07-2012 |
| | | WO | 2012092089 A1 | 05-07-2012 |
| | | WO | 2012092090 A1 | 05-07-2012 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7985722 B2
- EP 2074889 A1
- CN 102696880 A
- EP 3301196 A1
- WO 2018060517 A1

### Non-patent literature cited in the description

- *The EFSA Journal,* 2012, vol. 10 (1), 2536
- *Society of Nutrition Physiology,* 1999
- **ABDEL-MAWGOUD A.M. et al.** Rhamnolipids: diversity of structures, microbial origins and roles. *Appl Microbiol Biotechnol,* 2010, vol. 86, 1323-1336
- **ALI A.H. et al.** Rhamnolipids: Preparation, Determination and Applications - A Review. *American Journal of Food Science and Nutrition Research.,* 2017, vol. 4 (1), 9-17
- **CHONG H. ; LI Q.** Microbial production of rhamnolipids: opportunities, challenges and strategies. *Microb Cell Fact,* 2017, vol. 16, 137-149
- **DAMASCENO F.R.C. et al.** The combined use of a biosurfactant and an enzyme preparation to treat an effluent with a high fat content. *Colloids and Surfaces B: Biointerfaces,* 2012, vol. 95, 241-246
- EFSA Panel on Additives and Products or Substances used in Animal Feed (FEEDAP); Guidance for the preparation of dossiers for zootechnical additives. *EFSA Journal,* 2012, vol. 10 (1), 2536
- **IRORERE V.U. et al.** Microbial rhamnolipid production: a critical re-evaluation of published data and suggested future publication criteria. *Appl Microbiol Biotechnol,* 2017, vol. 101, 3941-3951
- **KHAFAGY E.S. et al.** Rhamnolipids enhance in vivo oral bioavailability of poorly absorbed molecules. *Pharm Res,* 2017
- **LIU G. et al.** Advances in applications of rhamnolipids biosurfactant in environmental remediation: A review. *Biotechnology & Bioengineering,* 2018, vol. 115 (4), 796-814
- **MAGALHAES L. ; NITSCHKE M.** Antimicrobial activity of rhamnolipids against Listeria monocytogenes and their synergistic interaction with nisin. *Food Control,* 2013, vol. 29, 138-142
- **MYERS W.D. et al.** Technical Note: A procedure for the preparation and quantitative analysis of samples for titanium dioxide. *J. Anim. Sci.,* 2004, vol. 82, 179-183
- **SINUMVAYO J.P ; ISHIMWE N.** Agriculture and food applications of rhamnolipids and its production by Pseudomonas Aeruginosa. *J Chem Eng Process Technol,* 2015, vol. 6 (2
- Empfehlungen zur Energie- und Nährstoffversorgung der Legehennen und Masthühner. Ausschuss für Bedarfsnormen der Gesellschaft für Ernährungsphysiologie. Society of Nutrition Physiology. DLG-Verlags-GmbH, 1999
- **VATSA P. et al.** Rhamnolipid biosurfactants as new players in animal and plant defense against microbes. *Int J Mol Sci.,* 2010, vol. 11, 5096-5109
- The chemical analysis of feedstuffs of VDLUFA. VDLUFA III. VDLUFA-Verlag, 1993